# EUROPEAN PATENT SPECIFICATION

(11) **EP 1 406 660 B1**
(45) Date of publication and mention of the grant of the patent: **06.06.2007**
(21) Application number: 02756521.7
(22) Date of filing: 18.07.2002
(51) Int. Cl.: A61K 45/06, A61K 31/585, A61K 31/44, A61K 31/40, A61K 31/365, A61K 31/22, A61P 9/00

(54) **COMBINATION OF AN ALDOSTERONE RECEPTOR ANTAGONIST AND AN HMG COA REDUCTASE INHIBITOR**
KOMBINATION VON EINEM ALDOSTERONE REZEPTOR ANTAGONISTEN UND EINEM HMG COA REDUKTASE HEMMER
COMBINAISON D'UN ANTAGONISTE DE RECEPTEUR D'ALDOSTERONE ET D'UN INHIBITEUR DE REDUCATSE D'HMG COA

(30) Priority: 19.07.2001 US 306336 P
(43) Date of publication of application: 14.04.2004
(73) Proprietor: Pharmacia Corporation, Peapack, NJ 07977 (US)
(72) Inventor: KELLER, Bradley, T., Chesterfield, MO 63017 (US); MCMAHON, Ellen, G., Sunset Hills, MO 63126 (US); ROCHA, Ricardo, Gurnee, IL 60031 (US)
(74) Representative: Boxall, Sarah Jane
(86) International application number: PCT/US2002/022896
(87) International publication number: WO 2003/007993

(56) References cited:
- WO-A-00/62775
- WO-A-98/47509
- WO-A-99/11260
- "CONTROVERSIES IN CARDIOLOGY AND CARDIAC SURGERY IN AFRICA" SAMJ. SOUTH AFRICAN MEDICAL JOURNAL - SAMT. SUID-AFRIKAANSE MEDIESE TIDSKRIF, PINELANDS, SA, vol. 91, no. 4, April 2001 (2001-04), page 290,292,294,296 XP001118752 ISSN: 0256-9574
- GARNETT W R: "INTERACTIONS WITH HYDROXYMETHYLGLUTARYL-COENZYME A REDUCTASE INHIBITORS" AMERICAN JOURNAL OF HEALTH-SYSTEM PHARMACY, XX, XX, vol. 52, 1 August 1995 (1995-08-01), pages 1639-1645, XP002941324 ISSN: 1079-2082
- "HERZINSUFFIZIENZ. THERAPIEKONZEPTE-WANDEL DURCH ERKENNTNISZUWACHS" THERAPIEWOCHE SCHWEIZ, THERAPIEWOCHE VERLAG AG, SENNWALD, CH, vol. 11, no. 10, 1995, page 482,484 XP000672381 ISSN: 0256-6869
- FOX K F: "A CRITICAL EVALUATION OF THE NICE GUIDELINES FOR POST-MYOCARDIAL INFARCTION PROPHYLAXIS" EXPERT OPINION ON PHARMACOTHERAPY, ASHLEY, LONDON,, GB, vol. 2, no. 12, 2001, pages 2079-2084, XP001117850 ISSN: 1465-6566
- PITT B ET AL: "THE EFFECT OF SPIRONOLACTIONE ON MORBIDITY AND MORTALITY IN PATIENTS WITH SEVERE HEART FAILURE" NEW ENGLAND JOURNAL OF MEDICINE, THE, MASSACHUSETTS MEDICAL SOCIETY, WALTHAM, MA, US, vol. 341, no. 10, 2 September 1999 (1999-09-02), pages 709-717, XP001069207 ISSN: 0028-4793
- LAMRINI R ET AL: "EFFETS INDESIRABLES ET INTERACTIONS MEDICAMENTEUSES DES HYPOLIPEMIANTS ADVERSE EFFECTS AND INTERACTION OF HYPOLIPIDAEMIC DRUGS" LYON PHARMACEUTIQUE, ELSEVIER, FR, vol. 48, no. 3, 1997, pages 142-151, XP001118757 ISSN: 0024-7804
- ALBERT M A ET AL: "EFFECT OF STATIN THERAPY ON C-REACTIVE PROTEIN LEVELS THE PRAVASTATIN INFLAMMATION/CRP EVALUATION (PRINCE): A RANDOMIZED TRIAL AND COHORT STUDY" JAMA THE JOURNAL OF THE AMERICAN MEDICAL ASSOCIATION, CHICAGO,IL, US, vol. 286, no. 1, 4 July 2001 (2001-07-04), pages 64-70, XP001119192 ISSN: 0098-7484
- GENTILE S ET AL: "COMPARATIVE EFFICACY STUDY OF ATORVASTATIN VS. SIMVASTATIN, PRAVASTATIN, LOVASTATIN AND PLACEBO IN TYPE 2 DIABETIC PATIENTS WITH HYPERCHOLESTEROLAEMIA" DIABETES, OBESITY AND METABOLISM, BLACKWELL SCIENCE, OXFORD, GB, vol. 2, 2000, pages 355-362, XP001094835 ISSN: 1462-8902
- MEIER C R ET AL: "HMG-COA REDUCTASE INHIBITORS AND THE RISK OF FRACTURES" JAMA THE JOURNAL OF THE AMERICAN MEDICAL ASSOCIATION, CHICAGO,IL, US, vol. 283, no. 24, 28 June 2000 (2000-06-28), pages 3205-3210, XP001119579 ISSN: 0098-7484
- MUNDY G ET AL: "STIMULATION OF BONE FORMATION IN VITRO AND IN RODENTS BY STATINS" SCIENCE, AMERICAN ASSOCIATION FOR THE ADVANCEMENT OF SCIENCE,, US, vol. 286, no. 5446, 3 December 1999 (1999-12-03), pages 1946-1949, XP000999135 ISSN: 0036-8075
- JICK H ET AL: "Statins and the risk of dementia" LANCET, XX, XX, vol. 356, no. 9242, 11 November 2000 (2000-11-11), pages 1627-1631, XP004264153 ISSN: 0140-6736

## Description

### BACKGROUND OF THE INVENTION

### Field of the Invention

The present invention relates to compounds suitable in the use for the treatment and/or prophylaxis of one or more pathogenic effects in a subject arising from or exacerbated by endogenous mineralocorticoid activity, especially in the presence of dyslipidemia or in a subject susceptible to or suffering from dyslipidemia. Particularly, the invention relates to the use of the aldosterone receptor antagonist, eplerenone, combined with the use of an HMG CoA reductase inhibitor for the treatment cardiovascular-related conditions.

### Description of the Related Art

### Aldosterone Receptor Antagonists

Aldosterone (ALDO) is the body's most potent known mineralocorticoid hormone. As connoted by the term mineralocorticoid, this steroid hormone has mineral-regulating activity. It promotes Na⁺ reabsorption not only in the kidney, but also from the lower gastrointestinal tract and salivary and sweat glands, each of which represents classic ALDO-responsive tissues. ALDO regulates Na⁺ and water resorption at the expense of potassium (K⁺) and magnesium (Mg²⁺) excretion.

ALDO can also provoke responses in nonepithelial cells. These responses can have adverse consequences on the structure and function of the cardiovascular system and other tissues and organs. Hence, ALDO can contribute to the organ failures for multiple reasons.

Multiple factors regulate ALDO synthesis and metabolism. These include renin as well as non-renin-dependent factors (such as K⁺, ACTH) that promote ALDO synthesis. Hepatic blood flow, by regulating the clearance of circulating ALDO, helps determine its plasma concentration, an important factor in heart failure characterized by reduction in cardiac output and hepatic blood flow.

The renin-angiotensin-aldosterone system (RAAS) is one of the hormonal mechanisms involved in regulating pressure/volume homeostasis and also in the development of hypertension. Activation of the renin-angiotensin-aldosterone system begins with renin secretion from the juxtaglomerular cells in the kidney and culminates in the formation of angiotensin II, the primary active species of this system. This octapeptide, angiotensin II, is a potent vasoconstrictor and also produces other physiological effects such as stimulating aldosterone secretion, promoting sodium and fluid retention, inhibiting renin secretion, increasing sympathetic nervous system activity, stimulating vasopressin secretion, causing positive cardiac inotropic effect and modulating other hormonal systems.

Previous studies have shown that antagonizing angiotensin II binding at its receptors is a viable approach to inhibit the renin-angiotensin system, given the pivotal role of this octapeptide which mediates the actions of the renin-angiotensin system through interaction with various tissue receptors. There are several known angiotensin II antagonists, both peptidic and non-peptidic in nature.

Many aldosterone receptor blocking drugs are known. For example, spironolactone is a drug that acts at the mineralocorticoid receptor level by competitively inhibiting aldosterone binding. This steroidal compound has been used for blocking aldosterone-dependent sodium transport in the distal tubule of the kidney in order to reduce edema and to treat essential hypertension and primary hyperaldosteronism [F. Mantero et al, Clin. Sci. Mol. Med., 45 (Suppl 1), 219s-224s (1973)]. Spironolactone is also used commonly in the treatment of other hyperaldosterone-related diseases such as liver cirrhosis and congestive heart failure. Progressively increasing doses of spironolactone from 1 mg to 400 mg per day [i.e., 1 mg/day, 5 mg/day, 20 mg/day] were administered to a spironolactone-intolerant patient to treat cirrhosis-related ascites [P.A. Greenberger et al, N. Eng. Reg. Allergy Proc., 7(4),343-345 (Jul-Aug, 1986)]. It has been recognized that development of myocardial fibrosis is sensitive to circulating levels of both Angiotensin II and aldosterone, and that the aldosterone antagonist spironolactone prevents myocardial fibrosis in animal models, thereby linking aldosterone to excessive collagen deposition [D. Klug et al, Am. J. Cardiol., 71 (3), 46A-54A (1993)]. Spironolactone has been shown to prevent fibrosis in animal models irrespective of the development of left ventricular hypertrophy and the presence of hypertension [C.G. Brilla et al, J. Mol. Cell. Cardiol., 25(5), 563-575 (1993)]. Spironolactone at a dosage ranging from 25 mg to 100 mg daily is used to treat diuretic-induced hypokalemia, when orally-administered potassium supplements or other potassium-sparing regimens are considered inappropriate [Physicians' Desk Reference, 55th Edn., p. 2971, Medical Economics Company Inc., Montvale, N.J. (2001)].

Previous studies have shown that inhibiting angiotensin converting enzyme (ACE) inhibits the renin-angiotensin system by substantially complete blockade of the formation of angiotensin II. Many ACE inhibitors have been used clinically to control hypertension. While ACE inhibitors may effectively control hypertension, side effects are common including chronic cough, skin rash, loss of taste sense, proteinuria and neutropenia.

Moreover, although ACE inhibitors effectively block the formation of angiotensin II, aldosterone levels are not well controlled in certain patients having cardiovascular diseases. For example, despite continued ACE inhibition in hypertensive patients receiving captopril, there has been observed a gradual return of plasma aldosterone to baseline levels [J. Staessen et al, J. Endocrinol., 91, 457-465 (1981)]. A similar effect has been observed for patients with myocardial infarction receiving zofenopril [C. Borghi et al, J. Clin. Pharmacol., 33 40-45 (1993)]. This phenomenon has been termed "aldosterone escape".

Another series of steroidal-type aldosterone receptor antagonists is exemplified by epoxy-containing spironolactone derivatives. For example, U.S. Patent No. 4,559,332 issued to Grob et al describes 9α,1 1α-epoxy-containing spironolactone derivatives as aldosterone antagonists useful as diuretics. These 9α, 11α-epoxy steroids have been evaluated for endocrine effects in comparison to spironolactone [M. de Gasparo et al, J. Pharm. Exp. Ther., 240(2), 650-656 (1987)].

Another series of steroidal-type aldosterone receptor antagonists is exemplified by drospirenone. Developed by Schering AG, this compound is a potent antagonist of mineralocorticoid and androgenic receptors, while also possessing progestagenic characteristics.

Combinations of an aldosterone antagonist and an ACE inhibitor have been investigated for treatment of heart failure. It is known that mortality is higher in patients with elevated levels of plasma aldosterone and that aldosterone levels increase as CHF progresses from activation of the Renin-Angiontensin-Aldosterone System (RAAS). Routine use of a diuretic may further elevate aldosterone levels. ACE inhibitors consistently inhibit angiotensin II production but exert only a mild and transient antialdosterone effect.

Combining an ACE inhibitor and spironolactone has been suggested to provide substantial inhibition of the entire RAAS. For example, a combination of enalapril and spironolactone has been administered to ambulatory patients with monitoring of blood pressure [P. Poncelet et al, Am. J. Cardiol., 65(2), 33K-35K (1990)]. In a 90-patient study, a combination of captopril and spironolactone was administered and found effective to control refractory CHF without serious incidents of hyperkalemia [U. Dahlstrom et al, Am. J. Cardiol., 71, 29A-33A (21 Jan 1993)]. Spironolactone coadministered with an ACE inhibitor was reported to be highly effective in 13 of 16 patients afflicted with congestive heart failure [A.A. van Vliet et al, Am. J. Cardiol., 71. 21A-28A (21 Jan 1993)]. Clinical improvements have been reported for patients receiving a co-therapy of spironolactone and the ACE inhibitor enalapril, although this report mentions that controlled trials are needed to determine the lowest effective doses and to identify which patients would benefit most from combined therapy [F. Zannad, Am. J. Cardiol., 71(3), 34A-39A (1993)]. In the Randomized Aldactone Evaluation Study, the effect of spironolactone and an ACE inhibitor were evaluated in 1663 patients with severe heart failure [B. Pitt, et al. NEJM 341(10):709-17 (1999)]. Results from this study showed a 30% reduction in mortality and a 35% reduction in hospitalizations, when spironolactone was added to ACE inhibitor therapy. A larger clinical study, EPHESUS, is currently underway to test the efficacy of eplerenone (epoxymexrenone), in combination with an ACE inhibitor, in over 6000 patients.

Combinations of an angiotensin II receptor antagonist and aldosterone receptor antagonist, are known. For example, PCT Application No. US91/09362 published 25 June 1992 describes treatment of hypertension using a combination of an imidazole-containing angiotensin II antagonist compound and spironolactone.

Combination therapies with an aldosterone antagonist may also be used as contraceptives. Combinations of drospirenone with estradiol (SH-641, Angeliq) and drospirenone with ethinyl estradiol (SH-470, Yasmin) are known. SH-470 is approved for use as an oral contraceptive.

### HMG-CoA Reductase Inhibitors

Numerous antihyperlipidemic agents having different modes of action have been disclosed in the literature as useful for the treatment of hyperlipidemic conditions and disorders. These agents include, for example, commercially available drugs such as nicotinic acid, bile acid sequestrants including cholestryramine and colestipol, 3-hydroxy-3-methylglutaryl coenzyme-A reductase inhibitors ("HMG Co-A reductase inhibitors" or "statins"), probucol, and fibric acid derivatives including gemfibrozil and clofibrate.

The class of antihyperlipidemic agents known as HMG Co-A reductase inhibitors operates by inhibiting the hepatic enzyme 3-hydroxy-3-methylglutaryl coenzyme-A reductase ("HMG Co-A reductase"). Direct inhibition of HMG Co-A reductase by the monotherapeutic administration of HMG Co-A reductase inhibitors such as pravastatin has been shown to be a clinically effective method of lowering serum LDL cholesterol. Sacks et al., "The Effect of Pravastatin on Coronary Events after Myocardial Infarction in Patients with Average Cholesterol Levels", New England Journal of Medicine, 335(14):1001-9 (1996). Monotherapeutic treatment with pravastatin may lead to upregulation of cell surface LDL receptors as a mechanism to provide cholesterol to the liver in support of bile acid synthesis. Fujioka et al., "The Mechanism of Comparable Serum Cholesterol Lowering Effects of Pravastatin Sodium, a 3-Hydroxy-3-Methylglutaryl Coenzyme A Inhibitor, between Once- and Twice-Daily Treatment Regimens in Beagle Dogs and Rabbits", Jpn. J. Pharmacol., Vol. 70, pp. 329-335 (1996).

The administration of an apical sodium-dependent bile acid transporter (ASBT) inhibitor in combination with an HMG Co-A reductase inhibitor is generally disclosed in PCT Application WO98/40375.

The treatment of hypercholesterolemia with an HMG Co-A reductase inhibitor in combination with a bile acid sequestering resin also has been reported in the literature. The administration of the HMG Co-A reductase inhibitor lovastatin in combination with the bile acid sequestering resin colestipol is disclosed in Vega et al., "Treatment of Primary Moderate Hypercholesterolemia With Lovastatin (Mevinolin) and Colestipol", JAMA, Vol. 257(1), pp. 33-38 (1987). The administration of the HMG Co-A reductase inhibitor pravastatin in combination with the bile acid sequestering resin cholestyramine is disclosed in Pan et al., "Pharmacokinetics and pharmacodynamics of pravastatin alone and with cholestyramine in hypercholesterolemia", Clin. Pharmacol. Ther., Vol. 48, No. 2, pp. 201-207 (August 1990). The administration of a combination therapy comprising a cholesterol ester transfer protein (CETP) inhibitor and a HMG Co-A reductase inhibitor is disclosed in U.S. Patent 5,932,587.

The treatment of hypercholesterolemia with other selected combination regimens also has been reported in the literature. Ginsberg, "Update on the Treatment of Hypercholesterolemia, with a Focus on HMG Co-A Reductase Inhibitors and Combination Regimens". Clin. Cardiol., Vol. 18(6), pp. 307-315 (June 1995), reports that, for resistant cases of hypercholesterolemia, therapy combining an HMG Co-A reductase inhibitor with either a bile acid sequestering resin, niacin or a fibric acid derivative generally is effective and well tolerated. Pasternak et al., "Effect of Combination Therapy with Lipid-Reducing Drugs in Patients with Coronary Heart Disease and 'Normal' Cholesterol Levels", Annals of Internal Medicine, Vol. 125, No. 7, pp. 529-540 (October 1, 1996) reports that treatment with either a combination of the HMG Co-A reductase inhibitor pravastatin and nicotinic acid or a combination of pravastatin and the fibric acid derivative gemfibrozil can be effective in lowering LDL cholesterol levels.

Some combination therapies for the treatment of cardiovascular disease have been described in the literature. Combinations of ASBT inhibitors with HMG CoA reductase inhibitors useful for the treatment of cardiovascular disease are disclosed in U.S. Patent Application No. 09/037,308.

A combination therapy of fluvastatin and niceritrol is described by J. Sasaki et al. (Id.). Those researchers conclude that the combination of fluvastatin with niceritrol "at a dose of 750 mg/day dose does not appear to augment or attenuate beneficial effects of fluvastatin."

L. Cashin-Hemphill et al. (J. Am. Med. Assoc., 264 (23), 3013-17 (1990)) describe beneficial effects of a combination therapy of colestipol and niacin on coronary atherosclerosis. The described effects include nonprogression and regression in native coronary artery lesions.

A combination therapy of acipimox and simvastatin shows beneficial HDL effects in patients having high triglyceride levels (N. Hoogerbrugge et al., J. Internal Med., 241. 151-55 (1997)).

Sitostanol ester margarine and pravastatin combination therapy is described by H. Gylling et al. (J. Lipid Res., 37, 1776-85 (1996)). That therapy is reported to simultaneously inhibit cholesterol absorption and lower LDL cholesterol significantly in non-insulin-dependent diabetic men.

Brown et al. (New Eng. J. Med., 323 (19), 1289-1339 (1990)) describe a combination therapy of lovastatin and colestipol which reduces atherosclerotic lesion progression and increase lesion regression relative to lovastatin alone.

A combination therapy of an apoB secretion inhibitor with a CETP inhibitor was disclosed by Chang et al. in PCT Patent Application No. WO 9823593.

Buch et al. (PCT Patent Application No. WO 9911263) describe a combination therapy comprising amlodipine and a statin compound for treating subjects suffering from angina pectoris, atherosclerosis, combined hypertension and hyperlipidemia, and to treat symptoms of cardiac arrest. Buch et al. describe in PCT Patent Application No. WO 9911259 a combination therapy comprising amlodipine and atorvastatin.

Scott et al. (PCT Patent Application No. WO 9911260) describe a combination therapy comprising atorvastatin and an antihypertensive agent.

Dettmar and Gibson (UK Patent Application No. GB 2329334 A) claim a therapeutic composition useful for reducing plasma low density lipoprotein and cholesterol levels, wherein the composition comprises an HMG CoA reductase inhibitor and a bile complexing agent.
The above references show continuing need to find safe, effective agents for the prophylaxis or treatment of diseases.

### Combination Therapy

Improved drug therapies, especially for patients who do not satisfactorily respond to conventional drug therapies, are highly desirable. Further, the increasing prevalence of such pathogenic effects, particularly cardiovascular-related conditions suggests that newer therapeutic interventions and strategies are needed to replace or complement current approaches. The present invention addresses this need and provides a new drug therapy comprising the administration of the aldosterone antagonist eplerenone combined with the use of one or more compounds that are HMG CoA reductase inhibitors, for the treatment of one or more of said pathogenic effects arising from or exacerbated by endogenous mineralocorticoid activity in a population of subjects characterized by or susceptible to dyslipidemia. Of interest are pathogenic effects arising from atherosclerosis, thus in one embodiment combination therapy would be used to prevent or treat myocardial infarction or stroke. In another embodiment combination therapy would be used to prevent or treat hypertension or heart failure or vascular disease. In another embodiment combination therapy would be used to prevent or treat renal dysfunction or end-organ damage. Such therapies are not limited to two components but may include one or more additional therapeutic compounds (e.g. a triple therapy) for treating the same or related disorders and provide some additional benefit to the patient.

The novel combinations of the present invention exhibit, for example, improved efficacy, improved potency, and/or reduced dosing requirements for the active compounds relative to therapeutic regimens previously disclosed in the published literature.

### SUMMARY OF THE INVENTION

Among the various aspects of the invention are:
1. Compounds suitable for the use in Methods for the treatment and/or prophylaxis of one or more pathogenic effects in a subject arising from or exacerbated by endogenous mineralocorticoid activity, wherein the method comprises administering therapeutically effective amounts of the aldosterone receptor antagonist eplerenone and a HMG CoA reductase inhibitor.
2. Compounds suitable for the use in methods for the treatment of cardiovascular-related conditions, comprising administering therapeutically effective amounts of the aldosterone receptor antagonist eplerenone and a HMG CoA reductase inhibitor.
3. The invention is farther directed to combinations, including pharmaceutical compositions, comprising the aldosterone receptor antagonist eplerenone and one or more HMG Co-A reductase inhibitors.
4. The invention is further directed to kits comprising the aldosterone receptor antagonist eplerenone and one or more HMG Co-A reductase inhibitors.
5. The invention is further directed to the preparation of a medicament, comprising the aldosterone receptor antagonist eplerenone and one or more HMG Co-A reductase inhibitors.

Other aspects of the invention will be in part apparent and in part pointed out hereinafter.

### DETAILED DESCRIPTION OF THE PREFERRED EMBODIMENTS

It has been discovered that the administration to a subject of the aldosterone receptor antagonist eplerenone and one or more HMG Co-A reductase inhibitors (particularly those HMG Co-A reductase inhibitors selected from the specific group consisting of compounds described below) provides improved results in the prophylaxis and/or treatment of one or more pathogenic effects in a subject arising from or exacerbated by endogenous mineralocorticoid activity, especially in the presence of dyslipidemia or in a subject susceptible to or suffering from dyslipidemia. Particularly, the invention relates to the use of the aldosterone receptor antagonist eplerenone combined with the use of an HMG CoA reductase inhibitor for the treatment of cardiovascular-related conditions. Of interest are pathogenic effects arising from atherosclerosis, thus in one embodiment combination therapy would be used to prevent or treat myocardial infarction or stroke or endothelial dysfunction. In another embodiment combination therapy would be used to prevent or treat hypertension or heart failure or left ventricular hypertrophy or vascular disease. In another embodiment combination therapy would be used to prevent or treat renal dysfunction or target-organ damage.

In a separate embodiment, one or more of said cardiovascular related conditions may be therapeutically or prophylacticaly treated with monotherapy, comprising administration of said aldosterone receptor antagonist eplerenone at a dose effective for treating or preventing said pathogenic effect.

### Aldosterone Receptor Antagonists

The term "aldosterone antagonist" denotes a compound capable of binding to an aldosterone receptor, as a competitive inhibitor of the action of aldosterone itself at the receptor site, so as to modulate the receptor-mediated activity of aldosterone. The superior selectivity of eplerenone results in a reduction in side effects, that can be caused by aldosterone antagonists that exhibit non-selective binding to non-mineralocorticoid receptors, such as androgen or progesterone receptors.

**TABLE I: Aldosterone Receptor Antagonist**

| Compound # | Structure | Name |
|---|---|---|
| 1 | 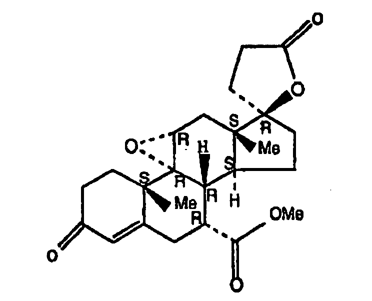 | Pregn-4-ene-7,21-dicarboxylic acid, 9,11-epoxy-17-hydroxy-3-oxo-,y-lactone, methyl ester, (7α,11α,17β)- |

Of particular interest is the compound eplerenone (also known as epoxymexrenone) which is compound 1 as shown above. Eplerenone is an aldosterone receptor antagonist and has a higher specificity for aldosterone receptors than does, for example, spironolactone. Selection of eplerenone as the aldosterone antagonist in the present method would be beneficial to reduce certain side-effects such as gynecomastia that occur with use of aldosterone antagonists having less specificity.

### HMG Co-A Reductase Inhibitors

The term "HMG Co-A reductase inhibitor" denotes a compound capable of reducing the rate of or completely blocking the reaction catalyzed by the enzyme HMG Co-A reductase. HMG Co-A reductase inhibitors encompassing a wide range of structures are useful in the combinations and methods of the present invention. Such HMG Co-A reductase inhibitors may be, for example, compounds that have been synthetically or semi-synthetically prepared, compounds extracted from natural sources such as plants, or compounds isolated as fungal metabolites from cultures of suitable microorganisms. Nonlimiting examples of HMG Co-A reductase inhibitors that may be used in the present invention include those HMG Co-A reductase inhibitors disclosed in Table 2, including the diastereomers, enantiomers, racemates, salts, tantomers, conjugate acids, and prodrugs of the HMG Co-A reductase inhibitors of Table 2. The therapeutic compounds of Table 2 can be used in the present invention in a variety of forms, including acid form, salt form, racemates, enantiomers, zwitterions, and tautomers.

**TABLE 2**

| **COMPOUNDS AND COMPOUND CLASSES** | **CAS NUMBERS FOR SPECIFIC AND REPRESENTATIVE COMPOUDS** | **REFERENCE** |
|---|---|---|
| Benfluorex | 23602-78-0 | ES 474498, Servier |
| Fluvastatin | 93957-54-1 | EP 244364, Sandoz |
| Lovastatin | 75330-75-5 | EP 22478, Merck & Co. |
| Pravastatin | 81093-37-0 | DE 3122499, Sankyo |
| Simvastatin | 79902-63-9 | EP 33538, Merck & Co. |
| Atorvastatin | 134523-00-5 | EP 409281, Warner-Lambert |
| Cerivastatin | 145599-86-6 | JP 08073-432, Bayer |
| Bervastatin and related benzopyrans | 132017-01-7 | EP 380392, Merck KGaA |
| ZD-9720 | | WO97/06802 |
| ZD-4522 (also called | 147098-20-2 (calcium salt); | EP 521471; |
| Rosuvastatin) | 147098-18-8 (sodium salt) | Bioorg. Med. Chem., Vol. 5(2), pp. 437-444 (1997); Drugs Future, Vol. 24 (5), pp. 511-513 (1999) |
| BMS 180431 | 129829-03-4; | Sit, Parker, Motoc, Han, |
| | 157243-11-3 | Balasubramanian, Catt, Brown, Harte, Thompson, and Wright, J. Med. Chem., (1990), 33(11), 2982-99; Bristol-Myers Squibb |
| NK-104 (also called pitavastatin and nisvastatin) | 141750-63-2 | Takano, Kamikubo, Sugihara, |
| | | Suzuk, Ogasawara, Tetahedron: Assymetry, (1993),4(2),201-4; Nissan Chemical |
| SR-12313 | 126411-39-0 | SmithKline Beecham |
| Carvastatin | 125035-66-7 | Tobishi Yakuhin Kogyo Co. Ltd. |
| PD-135022 | 122548-95-2 | Parke-Davis & Co. |
| Crilvastatin | 120551-59-9 | Pan Medica |
| (Carboxydihydroxy-heptenyl)- sulfonylpyrroles including S- 4522 | 148966-78-3,139993-44-5, | EP 464845; Shionogi |
| | 139993-45-6, 139993-46-7, | |
| | 139993-47-8, 139993-48-9, | |
| | 139993-49-0, 139993-50-3, | |
| | 139993-51-4, 139993-52-5, | |
| | 139993-53-6, 139993-54-7, | |
| | 139993-55-8, 139993-56-9, | |
| | 139993-57-0, 139993-58-1, | |
| | 139993-59-2, 139993-60-5, | |
| | 139993-61-6, 139993-62-7, | |
| | 139993-63-8, 139993-64-9, | |
| | 139993-65-0, 139993-66-1, | |
| | 139993-67-2, 139993-68-3, | |
| | 139993-69-4, 139993-70-7, | |
| | 139993-71-8, 139993-72-9, | |
| | 139993-73-0,139993-74-1, | |
| | 139993-75-2, 139993-76-3, | |
| | 139993-77-4, 139993-78-5, | |
| | 139993-79-6, 139993-80-9, | |
| | 140110-63-0, 140128-98-9, | |
| | 140128-99-0, 140157-62-6 | |
| Boron analogs of di- and tripeptides | 125894-01-1, 125894-02-2, | Sood, Sood Spielvogel, Hall, Eur. J. Med. Chem, (1990), 25(4), 301-8; Boron Biologicals |
| | 125894-03-3, 125894-04-4, | |
| | 125894-05-5, 125894-08-8, | |
| | 125894-09-9, 125914-96-7 | |
| Zaragozic Acids | 157058-13-4, 157058-14-5, | GB 2270312 |
| | 157058-15-6, 157058-16-7, | |
| | 157058-17-8.157058-18-9, | |
| | 157058-19-0 | |
| Seco-oxysterol analogs including U-88156 | 157555-28-7, 157555-29-8 | Larsen, Spilman, Yagi, Dith, Hart and Hess, J. Med. Chem, (1994), 37(15), 2343-51; Pharmacia & Upjohn |
| | | |
| | | |
| | | |
| U-9888; U-20685; U-51862; and U-71690 | 39945-32-9 | Pharmacia and Upjohn |
| Pyridopyrimidines including acitemate | 64405-40-9, | Hermecz, Meszaros, Vasvari- |
| | 101197-99-3 | Debreczy, Hovarth, Virag, and Sipos, Hung. Arzneim-Forsch., (1979), 29(12), 1833-5; Mitsubishi University |
| BMY 22566 | 129829-03-4 | Sit, Parker, Motoc, Han, Balasubramanian, Catt, Brown, Harte, Thompson, and Wright, J. Med. Chem., (1990), 33(11), 2982-99 |
| Colestolone | 50673-97-7 | Raulston, Mishaw, Parish and Schroepfer, Biochem. Biophys. Res. Commun., (1976), 71(4), 984-9; American Home Products |
| CP-83101 | 130746-82-6,130778-27-7 | Wint and McCarthy, J. Labelled Compd. Radiopharm., (1988), 25(11), 1289-97; Pfizer |
| Dalvastatin | 132100-55-1 | Kuttar, Windisch, Trivedi and Golebiowski, J. Chromatogr., A (1994), 678(2), 259-63; Rhone-Poulenc Rorer |
| Dihydromevinolin | 77517-29-4 | Falck and Yang, Tetrahedron Lett., (1984), 25(33), 3563-66; Merck & Co. |
| DMP-565 | 199480-80-3 | Ko, Trzaskos, Chen, Hauster, Brosz, and Srivastava, Abstr. Papers Am. Chem. Soc. (207^{th} National Meeting, Part 1, MEDI 10, 1994); Dupont Merck |
| ethenyldesmethyl-mevalonates including glenvastin | | Beck, Kessler, Baader, Bartmann, Bergmann, Granzer, Jendralla, Von Kerekjarto, Krause, et al., J. Med. Chem., (1990), 33(1), 52-60; Hoechst Marion Roussel |
| | | |
| | | |
| | | |
| | | |
| | | |
| | | |
| GR 95030 | 157243-22-6 | US 5316765; Glaxo Wellcome |
| Isoxazolopyridyl-mevalonates, | 130581-42-9, 130581-43-0, | EP 369323 |
| carboxylic acids and esters | 130581-44-1, 130581-45-2, | |
| | 130581-46-3, 130581-47-4, | |
| | 130581-48-5, 130581-49-6, | |
| | 130581-50-9, 130581-51-0, | |
| | 130581-52-1,130619-07-7, | |
| | 130619-08-8,130619-09-9 | |
| Lactones of 6-phenoxy-3,5-dihydroxy-hexanoic acids | 127502-48-1, 13606-66-1, 136034- | Jenderella, Granzer, Von Kerekjarto, Krause, Schnacht, Baader, Bartmann, Beck, Bergmann, et aL, J. Med. Chem, (1991), 34(10), 2962- 83; Hoechst Marion Roussel |
| | 04-3 | |
| | | |
| | | |
| | | |
| | | |
| L 659699 | 29066-42-0 | Chiang, Yang, Heck, Chabala, and Chang, J. Org. Chem, (1989), 54(24), 5708-12; Merck & Co. |
| | | |
| | | |
| | | |
| L 669262 | 130468-11-0 | Stokker, J. Org. Chem., (1994), 59(20). 5983-6; Merck & Co. |
| | | |
| | | |
| Mevastatin | 73573-88-3 | JP 56051992; Sankyo |
| Pannorin | 137023-81-5 | Ogawa, Hasumi, Sakai, Murzkwa and Endo, J. Antibiot. (1991), 44(7), 762- 7; Toyoko Noko University |
| | | |
| | | |
| | | |
| Rawsonol | 125111-69-5 | Cane, Troupe, Chan, Westley and Faulkner, Phytochemistry, (1989), 28(11), 2917-19; |
| | | |
| | | |
| | | SmithKline Beecham |
| RP 61969 | 126059-69-6 | EP 326386; Phone-Poulenc |
| | | Rorer |
| Bile Acid Derived HMG Co-A Reductase Inhibitors Including Na S-2467 and S-2468 | | Kramer, Wess, Enhsen, Bock, |
| | | Falk, Hofmann, Neckermann, |
| | | Grantz, Schulz, et aL, |
| | | Biochim. Biophys. Acta D, |
| | | (1994), 1227(3), 137-54; |
| | | Hoechst Marion Roussel |
| SC 32561 | 76752-41-5 | US 4230626; Monsanto |
| SC 45355 | 125793-76-2 | EP 329124; non-industrial source |
| | | |
| Phosphorus Containing HMG Co-A Reductase Inhibitors Including SQ 33600 | 133983-25-2 | US 5274155; Bristol-Myers |
| | | Squibb |
| | | |
| | | |
| 6-Aryloxymethyl-4- hydroxytetra-hydropyran-2- ones, carboxylic acids and salts | 135054-71-6, 136215-82-2, | EP 418648 |
| | 136215-83-3,136215-84-4, | |
| | 136215-85-5, 136315-18-9, | |
| | 136315-19-0,136315-20-3, | |
| | 136315-21-4, 136316-20-6 | |
| Atorvastatin calcium | 134523-03-8 | Baumann, Butler, Deering, |
| (CI 981) | | Mennen, Millar, Nanninga, |
| . | | Palmer and Roth, Tetrahedron |
| | | Lett., (1992), 33(17), 2283-4 |
| Mevinolin Analogs | | EP 245003 |
| Pyranone Derivatives | | US 4937259 |
| 1,2,4-Triazolidine-3,5-diones | 16044-43-2 | WO 9000897 |
| Isoazolidine-3,5-diones | 124756-24-7 | EP 321090 |
| CS-514 | 81181-70-6 | DE 3122499 |
| 1,10-bis(carboxy- | 32827-49-9 | DE 2038835 |
| methylthio)decane | | |
| α, β-, and γ- alkylaminophenone analogs including N-phenyl- piperazinopropio-phenone | | Huang and Hall, Eur. J. Med. |
| | | Chem., (1996),31(4),281-90 |
| | | |
| | | |
| 3-Amino-1-(2,3,4-mononitro-, mono- or dihalophenyl)- propan-1-ones including 3- morpholino-or piperidino-1- (3-nitrophenyl)-propan-1-ones | | Huang and Hall, Arch. Pharm., (1996), 329(7), 339-346 |
| | | |
| | | |
| | | |
| | | |
| Substituted isoxazolo | 64769-68-2 | US 4049813 |
| pyridinones | | |
| Biphenyl derivatives | | JP 07089898 |
| 4-[1-(Substituted phenyl)-2- oxo-pyrrolidin-4- yl]methoxybenzoic acids | | Watanabe, Ogawa, Ohno, |
| | | Yano, Yamada and Shirasaka, |
| | | Eur. J. Med. Chem., (1994), |
| | | 29(9), 675-86 |
| Dihydroxy(tetra-hydro- indazolyl, tetrahydrocyclo- pentapyrazolyl, or hexa- hydrocyclohepta-pyrazole)- heptenoate derivatives | | US 5134155 |
| | | |
| | | |
| | | |
| | | |
| HMG Co-A Reductase | | British Biotech & Japan |
| Inhibitors | | Tobacco |
| HMG Co-A Reductase | | Merck & Co. |
| Inhibitors | | |
| A-1233 | | Kitasato University |
| BAY-w-9533 | | Bayer |
| BB-476 | | British Biotech |
| BMS-180436 | | Bristol-Myers Squibb |
| BMY-22566 | | |
| HMG Co-A Reductase | | Bristol-Myers Squibb |
| Inhibitors | | |
| HMG Co-A Reductase | | Ono |
| Inhibitors | | |
| HMG Co-A Reductase | | Chiroscience |
| Inhibitors, Chiral | | |
| HMG Co-A Reductase | | Nissan Chemical |
| Inhibitors, isoxazolo-pyridine | | |
| HMG Co-A Reductase | | Pharmacia & Upjohn |
| Inhibitors, seco-oxysterol | | |
| HMG Co-A Reductase | | Sandoz |
| Inhibitors, thiophene | | |
| HMG Co-A Reductase | | Hoechest Marion Roussel |
| Inhibitors, 6-phenoxy-3,5- | | |
| dihydroxyhexanoic acids | | |
| Hypolipaemics | | Warner-Lambert |
| N-((1-methylpropyl)- carbonyl)-8- (2-(tetrahydro-4- hydroxy-6-oxo-2H-pyran-2- yl)ethyl)-perhydro- isoquinoline | | Sandoz |
| | | |
| | | |
| | | |
| | | |
| N-(1-oxododecyl)-4α,10- dimethyl-8-aza-trans-decal-3β- ol | | Hoechst Marion Roussel |
| | | |
| | | |
| P-882222 | | Nissan Chemical |
| S-853758A | | Hoechst Marion Roussel |
| (S)-4-((2-(4-(4-fluorophenyl)- 5-methyl-2-(1-methylethyl)-6- phenyl-3-pyridinyl)- ethenyl)hydroxy-phosphinyl)- 3-hydroxybutanoic acid, disodium salt | | Bristol-Myers Squibb |
| | | |
| | | |
| | | |
| | | |
| | | |
| SDZ-265859 | | Sandoz |
| (4R-(4α,6β(E)))-6-(2-(5-(4-fluorophenyl)-3-( 1-methyl-ethyl)- -1-(2-pyxidinyH-pyrazol-4-yl)ethenyl)tetra-hydro-4-hydroxy-2H-pyran-2-one | | Warner Lambert |
| 5β- aminoethyl-thiopentanoic acid derivatives | | Boehringer Mannheim |
| 6-amino-2-mercapto-5-methylpyrimidine-4-carboxylic acid | | North Carolina University |
| 6-phenoxymethyl- and 6-phenylethylen-(4-hydroxy-tetrahydropyran-2-one) analogues | | Hoechst Marion Roussel |

In one embodiment, the statin is selected from the group consisting of mevastatin, lovastatin, simvastatin, pravastatin, fluvastatin, atorvastatin, cerivastatin, bervastatin, ZD-4522 (also called rosuvastatin), BMS 180431, NK-104 (also called pitavastatin, nisvastatin, itavastatin), carvastatin, PD-135022, crilvastatin, acitemate, DMP-565, glenvastatin, L-659699, L-669262, S-2467, and S-2468.

In another embodiment, the statin is selected from the statins listed in Table 3 below. The individual patent documents referenced in Table 3 describe the preparation of these statins and are each herein incorporated by reference.

**TABLE 3**

| **Compound Number** | **Common Name** | **CAS Registry Number** | **Patent/Literature Reference for Preparation of Compound *Per Se*** |
|---|---|---|---|
| B-1 | Mevastatin | 73573-88-3 | U.S. 3,983,140 |
| B-2 | Lovastatin | 75330-75-5 | U.S. 4,231,938 |
| B-3 | Simvastatin | 79902-63-9 | U.S. 4,444,784 |
| B-4 | Pravastatin | 81093-37-0 | U.S. 4,346,227 |
| B-5 | Fluvastatin | 93957-54-1 | U.S. 4,739,073; U.S. 5,354,772 |
| B-6 | Atorvastatin | 134523-00-5 | EP 409281; U.S. 5,273,995 |
| B-7 | Cerivastatin | 145599-86-6 | U.S. 5,177,080 |
| B-8 | ZD-4522 (also called rosuvastatin) | 147098-20-2 | EP 521471, Example 7; Bioorg. Med. Chem., Vol. 5(2), pp. 437-444 (1997); Drugs Future, Vol. 24 (5), pp. 511-513 (1999) |
| B-9 | NK-104 (also called pitavastatin, nisvastatin, itavastatin) | 141750-63-2 | EP 0304063; CA 1336714 |

In another embodiment, the statin is selected from the group of statins consisting of lovastatin, simvastatin, pravastatin, atorvastatin, cerivastatin, ZD-4522 (also called rosuvastatin), and NK-104 (also called pitavastatin, nisvastatin, itavastatin).

In another embodiment, the statin is selected from the group of statins consisting of lovastatin, simvastatin, pravastatin, atorvastatin, and ZD-4522 (also called rosuvastatin).

In another embodiment, the statin is selected from the group of statins consisting of simvastatin, pravastatin, atorvastatin, and ZD-4522 (also called rosuvastatin).

In another embodiment, the statin is selected from the group of statins consisting of cerivastatin, ZD-4522 (also called rosuvastatin) and NK-104 (also called pitavastatin, nisvastatin, itavastatin).

In another embodiment, the statin is selected from the group of statins consisting of ZD-4522 (also called rosuvastatin) and NK-104 (also called pitavastatin, nisvastatin, itavastatin).

In another embodiment, the statin is selected from the group of statins consisting of lovastatin, simvastatin, pravastatin, and atorvastatin.

As noted above, the aldosterone receptor antagonists and HMG Co-A reductase inhibitors useful in the present combination therapy also may include the racemates and stereoisomers, such as diastereomers and enantiomers, of such inhibitors. Such stereoisomers can be prepared and separated using conventional techniques, either by reacting enantiomeric starting materials, or by separating isomers of compounds of the present invention. Isomers may include geometric isomers, for example cis isomers or trans isomers across a double bond. All such isomers are contemplated among the compounds of the present invention. Such isomers may be used in either pure form or in admixture with those inhibitors described above.

Furthermore, as also noted above, the aldosterone receptor antagonists and/or the HMG Co-A reductase inhibitors useful in the present combination therapy may be composed or formulated as prodrugs. The term "prodrug" includes a compound that is a drug precursor that, following administration to a subject and subsequent absorption, is converted to an active species *in vivo* via some process, such as metabolic conversion. Other products from the conversion process are easily disposed of by the body. More preferred prodrugs produce products from the conversion process that are generally accepted as safe. For example, the prodrug may be an acylated form of the active compound.

In addition to being particularly suitable for human use, the present combination therapy is also suitable for treatment of animals, including mammals such as horses, dogs, cats, rats, mice, sheep, pigs, and the like.

### Crystalline Forms of Active Compounds

It is particularly useful to select a form of each active compound that is easily handled, reproducible in form, easily prepared, stable and which is non-hygroscopic. By way of illustration and not limitation, several crystalline forms have been identified for the aldosterone antagonist eplerenone. These include Form H, Form L, various crystalline solvates and amorphous eplerenone. These forms, methods to make these forms and use of these forms in preparing compositions and medicaments, are disclosed in the following publications, incorporated herein by reference: WO 98/25948, WO 00/33847, WO 01/41535, WO 01/41770 and WO 01/42272.

### Definitions

The term "subject" as used herein refers to an animal, preferably a mammal, and particularly a human, who has been the object of treatment, observation or experiment.

The term "treatment" refers to any process, action, application, therapy, or the like, wherein a mammal, including a human being, is subject to medical aid with the object of improving the mammal's condition, directly or indirectly, including lessening the progression of a pathological effect.

The terms "prophylaxis" and "prevention" include either preventing the onset of a clinically evident pathological condition altogether or preventing the onset of a preclinically evident stage of a pathological condition in individuals. These terms encompass the prophylactic treatment of a subject at risk of developing a pathological condition.

The term "combination therapy" means the administration of two or more therapeutic agents to treat a pathological condition. Such administration encompasses coadministration of these therapeutic agents in a substantially simultaneous manner, such as in a single capsule having a fixed ratio of active ingredients or in multiple, separate capsules for each inhibitor agent. In addition, such administration encompasses use of each type of therapeutic agent in a sequential manner. In either case, the treatment regimen will provide beneficial effects of the drug combination in treating the pathological condition.

The phrase "therapeutically-effective" qualifies the amount of each agent that will achieve the goal of improvement in pathological condition severity and the frequency of incidence over treatment of each agent by itself, while avoiding adverse side effects typically associated with alternative therapies.

The term "pharmaceutically acceptable" is used adjectivally herein to mean that the modified noun is appropriate for use in a pharmaceutical product. Pharmaceutically acceptable cations include metallic ions and organic ions. More preferred metallic ions include, but are not limited to appropriate alkali metal salts, alkaline earth metal salts and other physiologically acceptable metal ions. Exemplary ions include aluminum, calcium, lithium, magnesium, potassium, sodium and zinc in their usual valences. Preferred organic ions include protonated tertiary amines and quaternary ammonium cations, including in part, trimethylamine, diethylamine, N,N'-dibenzylethylenediamine, chloroprocaine, choline, diethanolamine, ethylenediamine, meglumine (N-methylglucamine) and procaine. Exemplary pharmaceutically acceptable acids include without limitation hydrochloric acid, hydrobromic acid, phosphoric acid, sulfuric acid, methanesulfonic acid, acetic acid, formic acid, tartaric acid, maleic acid, malic acid, citric acid, isocitric acid, succinic acid, lactic acid, gluconic acid, glucuronic acid, pyruvic acid, oxalacetic acid, fumaric acid, propionic acid, aspartic acid, glutamic acid, benzoic acid, and the like. The specific salt(s) used will depend on the chemical structure of the active agent(s) in the pharmaceutical product. Methods for selecting pharmaceutically acceptable salts are well known in the pertinent art and can be found in standard text and reference books, such as the IUPAC Handbook of Pharmaceutical Salts. P. H. Stahl, et al., eds. (Wiley-VCH, 2002), incorporated herein by reference.

### Mechanism of Action

Without being held to a specific mechanism of action for the present combination therapy, it is hypothesized that the administration of these selected aldosterone receptor antagonists and HMG Co-A reductase inhibitors in combination is effective because of the simultaneous and interrelated responses of tissues and/or organs to these two distinct classes of drugs: marked down-regulation of aldosterone-stimulated genetic effects in response to the aldosterone antagonist and potent inhibition of de novo synthesis of cholesterol and various intermediates, in response to the HMG Co-A reductase inhibitor. A non-limiting example of an interrelated mechanism would be a decrease in aldosterone synthesis, via reduction of the aldosterone precursor cholesterol due to an HMG Co-A reductase inhibitor. Such an effect would provide a cooperative benefit to the therapeutic use of an aldosterone receptor antagonist. Another mechanism for therapeutic interactions between an aldosterone antagonist and a HMG Co-A reductase inhibitor could arise from anti-inflammatory effects of these drugs, in cooperation with reductions in serum LDL and hypertension, which would provide additional therapeutic benefit in treating or preventing atherosclerosis-related diseases.

### Advantages of Combination Therapy

The selected aldosterone receptor antagonists and HMG Co-A reductase inhibitors of the present invention act in combination to provide more than an additive benefit. For example, administration of an aldosterone receptor antagonist and HMG Co-A reductase inhibitor combination can result in the near-simultaneous reduction in pathogenic effects of multiple risk factors for atherosclerosis, such as high LDL levels, high aldosterone levels, high blood pressure, endothelial dysfunction, plaque formation and rupture, etc. can

The methods of this invention also provide for the effective prophylaxis and/or treatment of pathological conditions with reduced side effects compared to conventional methods known in the art. For example, administration of HMG Co-A reductase inhibitors can result in side effects such as, but not limited to, rhabdomyocytis, elevated liver enzymes, constipation, abdominal pain, dyspepsia, diarrhea, fever, flatulence, headache, myopathy, sinusitus, pharyngitis, myalgia, arthralgia, asthenia, and backpain. Rhabdomyocitis (muscle pain) and elevated liver enzymes (e.g., transaminases) occur more frequently at the highest recommended doses of most HMG Co-A reductase inhibitors. Reduction of the HMG Co-A reductase inhibitor doses in the present combination therapy below conventional monotherapeutic doses will minimize, or even eliminate, the side-effect profile associated with the present combination therapy relative to the side-effect profiles associated with, for example, monotherapeutic administration of HMG Co-A reductase inhibitors.

Periodic liver enzyme testing, typically every six months, is a routine procedure for subjects undergoing monotherapy with HMG Co-A reductase inhibitors. Because the present combination therapy minimizes or eliminates the presence of elevated liver enzymes, liver enzyme testing of subjects undergoing the present combination therapy may be discontinued or required at a much lower frequency than for HMG Co-A reductase inhibitor monotherapy. The side effects associated with the HMG Co-A reductase inhibitors typically are dose-dependent and, thus, their incidence increases at higher doses. Accordingly, lower effective doses of the HMG Co-A reductase inhibitors will result in fewer side effects than seen with higher doses of HMG Co-A reductase inhibitors in monotherapy or decrease the severity of such side-effects. In addition, the use of an aldosterone antagonist may provide a direct benefit in preventing or treating liver dysfunction, including ascites formation and hepatic fibrosis.

Other benefits of the present combination therapy include, but are not limited to, the use of a selected group of aldosterone receptor antagonists that provide a relatively quick onset of therapeutic effect and a relatively long duration of action. For example, a single dose of one of the selected aldosterone receptor antagonists may stay associated with the aldosterone receptor in a manner that can provide a sustained blockade of mineralocorticoid receptor activation. Another benefit of the present combination therapy includes, but is not limited to, the use of a selected group of aldosterone receptor antagonists, such as the epoxy-steroidal aldosterone antagonists exemplified by eplerenone, which act as highly selective aldosterone antagonists, with reduced side effects that can be caused by aldosterone antagonists that exhibit non-selective binding to non-mineralocorticoid receptors, such as androgen or progesterone receptors.

### Dosages and Treatment Regimen

### Aldosterone Receptor Antagonist Dosing

The amount of aldosterone antagonist that is administered and the dosage regimen for the methods of this invention depend on a variety of factors, including the age, weight, sex and medical condition of the subject, the severity of the pathogenic effect, the route and frequency of administration, and the particular aldosterone antagonist employed, and thus may vary widely. A daily dose administered to a subject of about 0.001 to 30 mg/kg body weight, or between about 0.005 and about 20 mg/kg body weight, or between about 0.01 and about 15 mg/kg body weight, or between about 0.05 and about 10 mg/kg body weight, or between about 0.01 to 5 mg/kg body weight, may be appropriate. The amount of aldosterone antagonist that is administered to a human subject typically will range from about 0.1 to 2000 mg, or from about 0.5 to 500 mg, or from about 0.75 to 250 mg, or from about 1 to 100 mg. A daily dose of aldosterone antagonist that produces no substantial diuretic and/or anti-hypertensive effect in a subject is specifically embraced by the present method. The daily dose can be administered in one to four doses per day.

Dosage unit forms of the pharmaceutical compositions can typically contain, for example, 10, 20, 25, 37.5, 50, 75, 100, 125, 150, 175, 200, 250, 300, 350 or 400 mg of the aldosterone receptor antagonist eplerenone. Preferred dosage unit forms contain about 25, 50, 100, or 150 mg of micronized eplerenone. The dosage unit form can be selected to accommodate the desired frequency of administration used to achieve the specified daily dosage. The amount of the unit dosage form of the pharmaceutical composition that is administered and the dosage regimen for treating the condition or disorder depends on a variety of factors, including the age, weight, sex and medical condition of the subject, the severity of the condition or disorder, the route and frequency of administration, and thus can vary widely, as is well known

Dosing of the aldosterone antagonist can be determined and adjusted based on measurement of blood pressure or appropriate surrogate markers (such as natriuretic peptides, endothelins, and other surrogate markers discussed below). Blood pressure and/or surrogate marker levels after administration of the aldosterone antagonist can be compared against the corresponding baseline levels prior to administration of the aldosterone antagonist to determine efficacy of the present method and titrated as needed. Non-limiting examples of surrogate markers useful in the method are surrogate markers for renal and cardiovascular disease.

### Prophylatic Dosing

It is beneficial to administer the aldosterone antagonist prophylatically, prior to a diagnosis of said inflammation-related cardiovascular disorders, and to continue administration of the aldosterone antagonist during the period of time the subject is susceptible to the inflammation-related cardiovascular disorders. Individuals with no remarkable clinical presentation but that are nonetheless susceptible to pathologic effects therefore can be placed upon a prophylatic dose of an aldosterone antagonist compound. Such prophylactic doses of the aldosterone antagonist may, but need not, be lower than the doses used to treat the specific pathogenic effect of interest.

### Cardiovascular Pathology Dosing

Dosing to treat pathologies of cardiovascular function can be determined and adjusted based on measurement of blood concentrations of natriuretic peptides. Natriuretic peptides are a group of structurally similar but genetically distinct peptides that have diverse actions in cardiovascular, renal, and endocrine homeostasis. Atrial natriuretic peptide ("ANP") and brain natriuretic peptide ("BNP") are of myocardial cell origin and C-type natriuretic peptide ("CNP") is of endothelial origin. ANP and BNP bind to the natriuretic peptide-A receptor ("NPR-A"), which, via 3',5'-cyclic guanosine monophosphate (cGMP), mediates natriuresis, vasodilation, renin inhibition, antimitogenesis, and lusitropic properties. Elevated natriuretic peptide levels in the blood, particularly blood BNP levels, generally are observed in subjects under conditions of blood volume expansion and after vascular injury such as acute myocardial infarction and remain elevated for an extended period of time after the infarction. (Uusimaa et al.: *Int. J. Cardiol 1999;* 69: 5-14).

A decrease in natriuretic peptide level relative to the baseline level measured prior to administration of the aldosterone antagonist indicates a decrease in the pathologic effect of aldosterone and therefore provides a correlation with inhibition of the pathologic effect. Blood levels of the desired natriuretic peptide level therefore can be compared against the corresponding baseline level prior to administration of the aldosterone antagonist to determine efficacy of the present method in treating the pathologic effect. Based upon such natriuretic peptide level measurements, dosing of the aldosterone antagonist can be adjusted to reduce the cardiovascular pathologic effect. Similarly, cardiac pathologies can also be identified, and the appropriate dosing determined, based on circulating and urinary cGMP Levels. An increased plasma level of cGMP parallels a fall in mean arterial pressure. Increased urinary excretion of cGMP is correlated with the natriuresis.

Cardiac pathologies also can be identified by a reduced ejection fraction or the presence of myocardial infarction or heart failure or left ventricular hypertrophy. Left ventricular hypertrophy can be identified by echo-cardiogram or magnetic resonance imaging and used to monitor the progress of the treatment and appropriateness of the dosing.

In another embodiment of the invention, therefore, the methods of the present invention can be used to reduce natriuretic peptide levels, particularly BNP levels, thereby also treating related cardiovascular pathologies.

### Renal Pathology Dosing

Dosing to treat pathologies of renal function can be determined and adjusted based on measurement of proteinuria, microalbuminuria, decreased glomerular filtration rate (GFR), or decreased creatinine clearance. Proteinuria is identified by the presence of greater than 0.3 g of urinary protein in a 24 hour urine collection. Microalbuminuria is identified by an increase in immunoassayable urinary albumin. Based upon such measurements, dosing of the aldosterone antagonist can be adjusted to reduce the renal pathologic effect.

### HMG Co-A Reductase Inhibitor Dosing

Dosage levels of the selected HMG Co-A reductase inhibitors useful in the present combination therapy typically are on the order of about 0.001 mg to about 1,000 mg daily, or levels of about 0.01 mg to about 500 mg daily, or levels of about 0.05 to about 100 mg daily. The preferred daily dosage of each HMG Co-A reductase inhibitor selected typically will be lower than the dosage recommended for conventional monotherapeutic treatment with that HMG Co-A reductase inhibitor. Examples of such conventionally recommended monotherapeutic dosages include about 10 to 80 mg for atorvastatin (for example, LiPITOR®); about 5 to 80 mg for simvastatin (for example, ZOCOR®); about 10 to 40 mg for pravastatin (for example, PRAVACHOL®); about 20 to 80 mg for lovastatin (for example, MEVACOR®); about 0.2 to 0.4 mg for cerivastatin (for example, BAYCOL®); and about 20 to 80 mg for fluvastatin (for example, LESCOL®).

It is understood, however, that the specific dose level for each patient will depend upon a variety of factors including the activity of the specific inhibitors employed, the age, body weight, general health, sex, diet, time of administration, rate of excretion, inhibitor combination selected, the severity of the particular conditions or disorder being treated, and the form of administration. Appropriate dosages can be determined in trials. The ratio of aldosterone receptor antagonist to HMG Co-A reductase inhibitor (weight/weight), however, typically will range from about 1:100 to about 100:1, or about 1:3 to about 50:1, or about 1:2 to about 20:1, or about 1:2 to about 10:1.

The total daily dose of each drug can be administered to the patient in a single dose, or in proportionate multiple subdoses. Subdoses can be administered two to six times per day. Doses can be in immediate release form or sustained release form effective to obtain desired results. Single dosage forms comprising the aldosterone receptor antagonist and the HMG Co-A reductase inhibitor may be used where desirable.

### Dosage Regimen

As noted above, the dosage regimen to prevent, treat, give relief from, or ameliorate a pathological condition, with the combinations and compositions of the present invention is selected in accordance with a variety of factors. These factors include the type, age, weight, sex, diet, and medical condition of the patient, the type and severity of the disease, the route of administration, pharmacological considerations such as the activity, efficacy, pharmacokinetics and toxicology profiles of the particular inhibitors employed, whether a drug delivery system is utilized, and whether the inhibitors are administered with other ingredients. Thus, the dosage regimen actually employed may vary widely and therefore deviate from the preferred dosage regimen set forth above.

Initial treatment of a patient suffering from a hyperlipidemic condition or disorder can begin with the dosages indicated above. Treatment generally should be continued as necessary over a period of several weeks to several months or years until the hyperlipidemic condition or disorder has been controlled or eliminated. Patients undergoing treatment with the combinations or compositions disclosed herein can be routinely monitored, for example in treating specific cardiovascular pathologies, by measuring blood pressure, ejection fraction, serum LDL or total cholesterol levels by any of the methods well-known in the art, to determine the effectiveness of the combination therapy. Continuous analysis of such data permits modification of the treatment regimen during therapy so that optimal effective amounts of each type of inhibitor are administered at any time, and so that the duration of treatment can be determined as well. In this way, the treatment regimen/dosing schedule can be rationally modified over the course of therapy so that the lowest amount of aldosterone receptor antagonist and HMG Co-A reductase inhibitor that together exhibit satisfactory effectiveness is administered, and so that administration is continued only so long as is necessary to successfully treat the hyperlipidemic condition.

In combination therapy, administration of the aldosterone receptor antagonist and the HMG Co-A reductase inhibitor may take place sequentially in separate formulations, or may be accomplished by simultaneous administration in a single formulation or separate formulations. Administration may be accomplished by any appropriate route, with oral administration being preferred. The dosage units used may with advantage contain one or more aldosterone receptor antagonist and one or more HMG Co-A reductase inhibitors in the amounts described above.

Dosing for oral administration may be with a regimen calling for a single daily dose, for multiple, spaced doses throughout the day, for a single dose every other day, for a single dose every several days, or other appropriate regimens. The aldosterone receptor antagonist and the HMG Co-A reductase inhibitor used in the combination therapy may be administered simultaneously, either in a combined dosage form or in separate dosage forms intended for substantially simultaneous oral administration. The aldosterone receptor antagonists and the HMG Co-A reductase inhibitors also may be administered sequentially, with either inhibitor being administered by a regimen calling for two-step ingestion. Thus, a regimen may call for sequential administration of the aldosterone receptor antagonist and the HMG Co-A reductase inhibitor with spaced-apart ingestion of these separate, active agents. The time period between the multiple ingestion steps may range from a few minutes to several hours, depending upon the properties of each active agent such as potency, solubility, bioavailability, plasma half-life and kinetic profile of the inhibitor, as well as depending upon the age and condition of the patient. Dose timing may also depend on the circadian or other rhythms for the pathological effects of agents, such as aldosterone, which may be optimally blocked at the time of their peak concentration. The combination therapy, whether administration is simultaneous, substantially simultaneous, or sequential, may involve a regimen calling for administration of the aldosterone receptor antagonist by oral route and the HMG Co-A reductase inhibitor by intravenous route. Whether these active agents are administered by oral or intravenous route, separately or together, each such active agent will be contained in a suitable pharmaceutical formulation of pharmaceutically acceptable excipients, diluents or other formulations components. Examples of suitable pharmaceutically - acceptable formulations are given above.

### Combinations and Compositions

The present invention is further directed to combinations, including pharmaceutical compositions, comprising the aldosterone receptor antagonist eplerenone and one or more HMG Co-A reductase inhibitors. In one embodiment, the present invention comprises a first amount of the aldosterone receptor antagonist, or a pharmaceutically acceptable salt, ester, or prodrug thereof; a second amount of the HMG Co-A reductase inhibitor, or a pharmaceutically acceptable salt, ester, conjugate acid, or prodrug thereof; and a pharmaceutically acceptable carrier. Preferably, the first and second amounts of the inhibitors together comprise a therapeutically effective amount of the inhibitors. The preferred aldosterone receptor antagonists and HMG Co-A reductase inhibitors used in the preparation of the compositions are as previously set forth above. The combinations and compositions comprising an aldosterone receptor antagonist and an HMG Co-A reductase inhibitor of the present invention can be administered for the prophylaxis and/or treatment of pathological conditions, as previously set forth, by any means that produce contact of these inhibitors with their site of action in the body.

For the prophylaxis or treatment of the pathological conditions referred to above, the combination administered can comprise the inhibitor compounds *per se.* Alternatively, pharmaceutically acceptable salts are particularly suitable for medical applications because of their greater aqueous solubility relative to the parent compound.

The combinations of the present invention also can be presented with a pharmaceutically acceptable carrier in the form of a pharmaceutical composition. The carrier must be acceptable in the sense of being compatible with the other ingredients of the composition and must not be deleterious to the recipient. The carrier can be a solid or a liquid, or both, and preferably is formulated with the compound as a unit-dose composition, for example, a tablet, which can contain from 0.05% to 95% by weight of the active compounds. Other pharmacologically active substances can also be present, including other compounds useful in the present invention. The pharmaceutical compositions of the invention can be prepared by any of the well-known techniques of pharmacy, such as admixing the components.

The combinations and compositions of the present invention can be administered by any conventional means available for use in conjunction with pharmaceuticals. Oral delivery of the aldosterone receptor antagonist and the HMG Co-A reductase inhibitor is generally preferred (although the methods of the present invention are still effective, for example, if the HMG Co-A reductase inhibitor is administered parenterally). The amount of each inhibitor in the combination or composition that is required to achieve the desired biological effect will depend on a number of factors including those discussed below with respect to the treatment regimen.

Orally administrable unit dose formulations, such as tablets or capsules, can contain, for example, from about 0.1 to about 2000 mg, or about 0.5 mg to about 500 mg, or from about 0.75 to about 250 mg, or from about 1 to about 100 mg of the aldosterone receptor antagonist, and/or from about 0.01 to about 500 mg, or about 0.75 mg to about 100 mg, or from about 0.1 to about 50 mg, of the HMG Co-A reductase inhibitor.

Oral delivery of the aldosterone receptor antagonist and the HMG Co-A reductase inhibitors of the present invention can include formulations, as are well known in the art, to provide immediate delivery or prolonged or sustained delivery of the drug to the gastrointestinal tract by any number of mechanisms. Immediate delivery formulations include, but are not limited to, oral solutions, oral suspensions, fast-dissolving tablets or capsules, disintegrating tablets and the like. Prolonged or sustained delivery formulations include, but are not limited to, pH sensitive release from the dosage form based on the changing pH of the small intestine, slow erosion of a tablet or capsule, retention in the stomach based on the physical properties of the formulation, bioadhesion of the dosage form to the mucosal lining of the intestinal tract, or enzymatic release of the active drug from the dosage form. The intended effect is to extend the time period over which the active drug molecule is delivered to the site of action by manipulation of the dosage form. Thus, enteric-coated and enteric-coated controlled release formulations are within the scope of the present invention. Suitable enteric coatings include cellulose acetate phthalate, polyvinylacetate phthalate, hydroxypropylmethyl-cellulose phthalate and anionic polymers of methacrylic acid and methacrylic acid methyl ester.

Pharmaceutical compositions suitable for oral administration can be presented in discrete units, such as capsules, cachets, lozenges, or tablets, each containing a predetermined amount of at least one compound of the present invention; as a powder or granules; as a solution or a suspension in an aqueous or non-aqueous liquid; or as an oil-in-water or water-in-oil emulsion. As indicated, such compositions can be prepared by any suitable method of pharmacy which includes the step of bringing into association the inhibitor(s) and the carrier (which can constitute one or more accessory ingredients). In general, the compositions are prepared by uniformly and intimately admixing the inhibitor(s) with a liquid or finely divided solid carrier, or both, and then, if necessary, shaping the product. For example, a tablet can be prepared by compressing or molding a powder or granules of the inhibitors, optionally with one or more assessory ingredients. Compressed tablets can be prepared by compressing, in a suitable machine, the compound in a free-flowing form, such as a powder or granules optionally mixed with a binder, lubricant, inert diluent and/or surface active/dispersing agent(s). Molded tablets can be made, for example, by molding the powdered compound in a suitable machine.

Liquid dosage forms for oral administration can include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

Pharmaceutical compositions suitable for buccal (sub-lingual) administration include lozenges comprising a compound of the present invention in a flavored base, usually sucrose, and acacia or tragacanth, and pastilles comprising the inhibitors in an inert base such as gelatin and glycerin or sucrose and acacia.

In any case, the amount of aldosterone receptor antagonist and HMG Co-A reductase inhibitor that can be combined with carrier materials to produce a single dosage form to be administered will vary depending upon the host treated and the particular mode of administration. The solid dosage forms for oral administration including capsules, tablets, pills, powders, and granules noted above comprise the inhibitors of the present invention admixed with at least one inert diluent such as sucrose, lactose, or starch. Such dosage forms may also comprise, as in normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Pharmaceutically acceptable carriers encompass all the foregoing and the like. The above considerations in regard to effective formulations and administration procedures are well known in the art and are described in standard textbooks. Formulation of drugs is discussed in, for example, Hoover, John E., Remington's Pharmaceutical Sciences, Mack Publishing Co., Easton, Pennsylvania, 1975; Liberman, et al., Eds., Pharmaceutical Dosage Forms, Marcel Decker, New York, N.Y.,1980; and Kibbe, et al., Eds., Handbook of Pharmaceutical Excipients (3^{rd} Ed.), American Pharmaceutical Association, Washington, 1999.

**TABLE 4: EXAMPLES OF COMBINATION THERAPIES**

| **ALDOSTERONE RECEPTOR ANTAGONIST** | **HMG CO-A REDUCTASE INHIBITOR (COMPOUND NUMBER-TABLE 3)** |
|---|---|
| Eplerenone | B-1 |
| Eplerenone | B-2 |
| Eplerenone | B-3 |
| Eplerenone | B-4 |
| Eplerenone | B-5 |
| Eplerenone | B-6 |
| Eplerenone | B-7 |
| Eplerenone | B-8 |
| Eplerenone | B-9 |

### Kits

The present invention further comprises kits that are suitable for use in performing the methods of treatment and/or prophylaxis described above. In one embodiment, the kit contains a first dosage form comprising one or more of the aldosterone receptor antagonists previously identified and a second dosage form comprising an HMG Co-A reductase inhibitor identified in Table 2 or Table 3 in quantities sufficient to carry out the methods of the present invention. Preferably, the first dosage form and the second dosage form together comprise a therapeutically effective amount of the inhibitors for the prophylaxis and/or treatment of a pathological condition. In another embodiment, the kit contains a first dosage form comprising the aldosterone receptor antagonist eplerenone and a second dosage form comprising an HMG Co-A reductase inhibitor. In a preferred embodiment, the kit contains a first dosage form comprising the aldosterone receptor antagonist eplerenone and a second dosage form comprising an HMG Co-A reductase inhibitor identified in Table 2. In a more preferred embodiment, the kit contains a first dosage form comprising the aldosterone receptor antagonist eplerenone and a second dosage form comprising an HMG Co-A reductase inhibitor identified in Table 3. In another embodiment, the kit contains a first dosage form comprising the aldosterone receptor antagonist spironolactone and a second dosage form comprising an HMG Co-A reductase inhibitor. In a preferred embodiment, the kit contains a first dosage form comprising the aldosterone receptor antagonist spironolactone and a second dosage form comprising an HMG Co-A reductase inhibitor identified in Table 2. In a more preferred embodiment, the kit contains a first dosage form comprising the aldosterone receptor antagonist spironolactone and a second dosage form comprising an HMG Co-A reductase inhibitor identified in Table 3.

The following nonlimiting examples serve to illustrate various aspects of the present invention.

### EXAMPLE 1: THERAPEUTIC TREATMENT

Numerous well known, in vitro and in vivo testing schemes and protocols are useful to demonstrate the efficacy of aldosterone receptor antagonists and HMG Co-A reductase inhibitors, both separately and in combination, for treating or preventing said pathogenic effects. Non-limiting examples of testing schemes and protocols are described in references listed below, which are incorporated herein by reference.
Pitt, et al. NEJM 341, 709-717 (1999)
Pitt, et al. Cardiovasc Drug Ther 15:79-87 (2001)
De Gasparo, et al. J Pharm Exp Ther 240, 650-656 (1986)
Blazer-Yost, et al. Am. J. Physiol 272, C1928-C1935 (1997)
Vijan, et al. J Gen Intern Med 12, 567-580 (1997)
Gentile, et al. Diabetes, Obesity and Metabolism 2, 355-362 (2000)
Sheng-Fang, et al. Am J Cardiol 86, 514-518 (2000)
Jick, et al. Lancet 356, 1627-1631 (2000)
Albert, et al. JAMA 286, 64-70 (2001)
Ridker, et al. NEJM 344, 1959-1965 (2001)
Wang, et al. JAMA 283, 3211-3216 (2000)
Meier, et al. JAMA 283, 3205-3210 (2000)
Sugiyama, et al. Biochem Biophys Res Commun 271, 688-692 (2000)
Mundy, et al. Science 286, 1946-1949 (1999)
Xiao, et al. J Endocrinol 165, 533-536 (2000)
US Patent 5,730,992, US Patent 5,932,587, US Patent 6,180,597
WO 00/69446, WO 00/69445, WO 00/45818, WO 00/45817, WO 99/66930,
WO 99/11260, WO 01/34132, WO 00/51642

### EXAMPLE 2: COMPOSITIONS

The combinations and compositions of the present invention can be administered by any conventional means available for use in conjunction with pharmaceuticals. Oral delivery of the aldosterone receptor antagonist and the HMG Co-A reductase inhibitor is generally preferred (although the methods of the present invention are still effective, for example, if the HMG Co-A reductase inhibitor is administered parenterally). The amount of each inhibitor in the combination or composition that is required to achieve the desired biological effect will depend on a number of factors including including patrients age, weight and physical/medical status. Non-limiting examples of pharmaceutical compositions are described in references listed below, which are incorporated herein by reference.
WO O1/41770, WO 00/33847

### EXAMPLE 3: PHARMACEUTICAL COMPOSITIONS

120 mg tablets having the composition set forth in Table X-1 can be prepared using wet granulation techniques:

**TABLE X-1**

| **INGREDIENT** | **WEIGHT (mg)** |
|---|---|
| Eplerenone | 25 |
| Pravastatin | 20 |
| Lactose | 54 |
| Microcrystalline Cellulose | 15 |
| Hydroxypropyl Methyl Cellulose | 3 |
| Croscarmellose Sodium | 2 |
| Magnesium Stearate | 1 |
| Total Tablet Weight | 120 |

### EXAMPLE 4: PHARMACEUTICAL COMPOSITIONS

120 mg tablets having the composition set forth in Table X-2 can be prepared using direct compression techniques:

**TABLE X-2**

| **INGREDIENT** | **WEIGHT FRACTION (mg)** |
|---|---|
| Eplerenone | 25 |
| Pravastatin | 5 |
| Lactose | 69.5 |
| Microcrystalline Cellulose | 15 |
| Colloidal Silicon Dioxide | 0.5 |
| Talc | 2.5 |
| Croscarmellose Sodium | 2 |
| Magnesium Stearate | 0.5 |
| Total Tablet Weight | 120 |

### EXAMPLE 5: PHARMACEUTICAL COMPOSITIONS

120 mg tablets having the composition set forth in Table X-3 can be prepared using wet granulation techniques:

**TABLE X-3**

| **INGREDIENT** | **WEIGHT (mg)** |
|---|---|
| Eplerenone | 25 |
| Simvastatin | 20 |
| Lactose | 54 |
| Microcrystalline Cellulose | 15 |
| Hydroxypropyl Methyl Cellulose | 3 |
| Croscarmellose Sodium | 2 |
| Magnesium Stearate | 1 |
| Total Tablet Weight | 120 |

### EXAMPLE 6: PHARMACEUTICAL COMPOSITIONS

120 mg tablets having the composition set forth in Table X-4 can be prepared using direct compression techniques:

**TABLE X-4**

| **INGREDIENT** | **WEIGHT FRACTION (mg)** |
|---|---|
| Eplerenone | 25 |
| Simvastatin | 5 |
| Lactose | 69.5 |
| Microcrystalline Cellulose | 15 |
| Colloidal Silicon Dioxide | 0.5 |
| Talc | 2.5 |
| Croscarmellose Sodium | 2 |
| Magnesium Stearate | 0.5 |
| Total Tablet Weight | 120 |

### EXAMPLE 7: PHARMACEUTICAL COMPOSITIONS

120 mg tablets having the composition set forth in Table X-5 can be prepared using wet granulation techniques:

**TABLE X-5**

| **INGREDIENT** | **WEIGHT (mg)** |
|---|---|
| Eplerenone | 25 |
| Atorvastatin | 10 |
| Lactose | 64 |
| Microcrystalline Cellulose | 15 |
| Hydroxypropyl Methyl Cellulose | 3 |
| Croscarmellose Sodium | 2 |
| Magnesium Stearate | 1 |
| Total Tablet Weight | 120 |

### EXAMPLE 8: PHARMACEUTICAL COMPOSITIONS

105 mg tablets having the composition set forth in Table X-6 can be prepared using direct compression techniques:

**TABLE X-6**

| **INGREDIENT** | **WEIGHT FRACTION (mg)** |
|---|---|
| Eplerenone | 10 |
| Atorvastatin | 2.5 |
| Lactose | 72 |
| Microcrystalline Cellulose | 15 |
| Colloidal Silicon Dioxide | 0.5 |
| Talc | 2.5 |
| Croscarmellose Sodium | 2 |
| Magnesium Stearate | 0.5 |
| Total Tablet Weight | 105 |

### EXAMPLE 9: PREPARATION OF ALDOSTERONE RECEPTOR ANTAGONISTS AND HMG CO-A REDUCTASE INHIBITORS

Procedures for synthesis of HMG Co-A reductase inhibitors are well known and described in numerous published documents. Non-limiting examples of synthetic schemes and protocols are described in references listed below, which are incorporated herein by reference.

### HMG Co-A Reductase inhibitors:

ES 474498 EP 244364 EP 22478, DE 3122499, EP 33538,
EP 409281, JP 08073-432, EP 380392, WO 97/06902, EP 521471,
Bioorg. Med. Chem. 5(2), pp. 437-444 (1997)
Drugs Future 24 (5), pp. 511-513 (1999)
J. Med. Chem 33(11), 2982.99 (1990)
Tetahedron: Assymetry 4(2), 201-4 (1993)

### EXAMPLE 10: PHYSICAL FORMS OF ALDOSTERONE RECEPTOR ANTAGONISTS AND HMG CO-A REDUCTASE INHIBITORS IN MEDICAMENTS

It is particularly useful to select a form of each active compound that is easily handled, reproducible in form, easily prepared, stable and which is non-hygroscopic. By way of illustration and not limitation, several crystalline forms have been identified for the aldosterone antagonist eplerenone. These include Form H, Form L, various crystalline solvates and amorphous eplerenone. These forms, methods to make these forms and use of these forms in preparing compositions and medicaments, are disclosed in the following publications, incorporated herein by reference: WO 98/25948, WO 00/33847, WO 01/41535, WO 01/41770 and WO 01/42272.

### EXAMPLE 11: CLINICAL EVENTS TRIAL

The following is a description of a clinical trial employing a co-therapy of an aldosterone receptor antagonist and an HMG CoA reductase inhibitor to exemplify the methods of the present invention.

This is a primary prevention endpoint event trial. Inclusion criteria are LDL-cholesterol 130-190 mg/dl (or <130 if the ratio of total cholesterol/HDL is >6) and HDL-cholesterol <45 mg/dl. The trial is designed to study the effect of co-therapy of an aldosterone receptor antagonist and an HMG CoA reductase inhibitor in a cohort with average to mildly elevated LDL-cholesterol and a below average HDL-cholesterol.

This is a double-blind, randomized, placebo controlled trial designed and powered to investigate whether co-therapy of an aldosterone receptor antagonist and an HMG CoA reductase inhibitor will decrease the rate of first acute major coronary events (e.g. sudden cardiac death, fatal and non-fatal myocardial infarction and unstable angina) compared to intervention with an HMG CoA reductase inhibitor alone. Secondary objectives include whether co-therapy treatment, compared to HMG CoA reductase inhibitor alone, will decrease cardiovascular morbidity and mortality across the spectrum of clinical events, by measuring the rates of: (1) fatal and non-fatal coronary revascularization procedures (2) unstable angina, (3) fatal and non-fatal myocardial infarction, (4) fatal and non-fatal cardiovascular events, (5) fatal and non-fatal coronary events.

A four-week HMG CoA reductase inhibitor alone baseline run-in is followed by randomization of participants to additional treatment with an aldosterone receptor antagonist, such as eplerenone, or placebo.

Baseline measurements at randomization include lipid analysis (including Apo Al and Apo B), hematology, blood chemistry and urinalysis.

During the first year of active treatment, participants returne to clinic at 4 week intervals. At each visit, participants are asked about adverse events and undergo laboratory safety tests for liver enzymes, creatine kinase and an extensive evaluation that includes a physical exam, electrocardiogram, mammography (women), ophthalmological examination, complete blood chemistry, hematology and urinalysis.

All subjects are followed until the decision to end the study after a median duration of 4 years of treatment. The trial design for the final analysis provides sufficient power to detect the reductions in the number of patients experiencing any of the following:
Primary Endpoints:
   1 - acute major coronary events defined as fatal and non-fatal myocardial infarction
   2 - unstable angina
   3 - sudden cardiac death
Secondary Endpoints:
   1 - revascularizations
   2 - unstable angina
   3 - fatal and nonfatal MI
   4 - fatal and nonfatal cardiovascular events
   5 - fatal and nonfatal coronary events

### EXAMPLE 12: EVALUATION OF CORONARY/CAROTID ARTERY DISEASE

The utility of the co-therapy of the present invention in treating atherosclerosis is demonstrated in the clinical trial protocol described below.

This study is a prospective double-blind, placebo-controlled trial of the effect of a combination of an aldosterone receptor antagonist and an HMG CoA reductase inhibitor on the progression/regression of existing coronary artery disease as evidenced by changes in coronary angiography or carotid ultrasound.

Entry criteria: Subjects must be adult male or female, aged 18-80 years of age in whom coronary angiography is clinically indicated. Subjects will have angiographic presence of a significant focal lesion such as 30% to 50% on subsequent evaluation by quantitative coronary angiography (QCA) in a minimum of one segment. Segments to be analyzed include: left main, proximal, mid and distal left anterior descending, first and second diagonal branch, proximal and distal left circumflex, proximal, mid and distal right coronary artery.

At entry subjects undergo quantitative coronary angiography, B-mode carotid artery ultrasonography and assessment of carotid arterial compliance. Subjects are randomized to receive an aldosterone receptor antagonist and placebo, or an HMG CoA reductase inhibitor and placebo, or co-therapy of an aldosterone receptor antagonist and an HMG CoA reductase inhibitor. Subjects are monitored for three years. B- mode carotid ultrasound assessment of carotid artery atherosclerosis and compliance are performed at regular intervals throughout the study.

Coronary angiography is performed at the end of the three year period. Baseline and post-treatment angiograms and the intervening carotid artery B-mode ultrasonograms are evaluated for new lesions or progression of existing atherosclerotic lesions. Arterial compliance measurements are assessed for changes from baseline.

The primary objective of this study is to show that the co-therapy of an aldosterone receptor antagonist and an HMG CoA reductase inhibitor reduces the progression of atherosclerotic lesions as measured by quantitative coronary angiography (QCA) in subjects with clinical coronary artery disease.

The primary endpoint of the study is the change in the average mean segment diameter of coronary arteries.

The secondary objective of this study is to demonstrate that the combination of an aldosterone receptor antagonist and an HMG CoA reductase inhibitor reduces the rate of progression of atherosclerosis in the carotid arteries as measured by the slope of the maximum intimal-medial thickness measurements averaged over 12 separate wall segments (Mean Max) as a function of time, more than does an HMG CoA reductase inhibitor or an aldosterone receptor antagonist alone.

The examples herein can be performed by substituting the generically or specifically described reactants and/or operating conditions of this invention for those used in the preceding examples.

In view of the above, it will be seen that the several objects of the invention are achieved. As various changes could be made in the above methods, combinations and compositions of the present invention without departing from the scope of the invention, it is intended that all matter contained in the above description be interpreted as illustrative and not in a limiting sense. All documents mentioned in this application are expressly incorporated by reference as if fully set forth at length.

When introducing elements of the present invention or the preferred embodiment(s) thereof, the articles "a", "an", "the" and "said" are intended to mean that there are one or more of the elements. The terms "comprising", "including" and "having" are intended to be inclusive and mean that there may be additional elements other than the listed elements.

## Claims

1. A combination comprising a first amount of eplerenone and a second amount of an HMG Co-A reductase inhibitor.

2. A combination according to Claim 1 wherein the combination is a combined dosage form of eplerenone and an HMG Co-A reductase inhibitor.

3. A combination according to Claim 1 wherein the combination is a kit comprising eplerenone and an HMG Co-A reductase inhibitor.

4. A combination according to Claim 1 wherein the combination is in the form of a pharmaceutical composition further comprising a pharmaceutically acceptable carrier.

5. A combination according to Claim 4 wherein said first amount and said second amount together comprise a therapeutically-effective amount of said eplerenone and HMG Co-A reductase inhibitor.

6. A combination according to any one of Claims 1 to 5 wherein said HMG Co-A reductase inhibitor is selected from the group consisting of mevastatin, lovastatin, simvastatin, pravastatin, fluvastatin, cerivastatin, atorvastatin, rosuvastatin, pitavastatin, and the pharmaceutically acceptable salts, esters, conjugate acids, and prodrugs thereof.

7. A combination according to any one of Claims 1 to 5 wherein said HMG Co-A reductase inhibitor is atorvastatin or a pharmaceutically acceptable salt, ester, conjugate acid, or prodrug thereof.

8. A combination according to any one of Claims 1 to 7 wherein said eplerenone and said HMG Co-A reductase inhibitor are present in said composition in a weight ratio range from about ten-to-one to about one-to-two of said eplerenone to said HMG Co-A reductase inhibitor.

9. A combination according to Claim 8 wherein said weight ratio range is from about five-to-one to about one-to-one.

10. A combination according to Claim 8 wherein said weight ratio range is from about two-to-one to about one-to-one.

11. A combination according to any one of Claims 1 to 10 wherein said second amount of said HMG Co-A reductase inhibitor is between about 0.05 mg to about 100 mg.

12. A combination according to any one of Claims 1 to 11 wherein said first amount of said eplerenone is between about 0.75 mg to about 200 mg.

13. Use of eplerenone and an HMG Co-A reductase inhibitor for the manufacture of a medicament for simultaneous or sequential use in the treatment or prevention of a cardiovascular condition.

14. Use according to Claim 13 wherein said cardiovascular condition is selected from the group consisting of atherosclerosis, hypertension, heart failure, vascular disease, renal dysfunction, stroke, myocardial infarction, endothelial dysfunction, ventricular hypertrophy, target-organ damage, thrombosis, cardiac arrhythmia, plaque rupture and aneurysm.

15. Use according to Claim 13 or Claim 14 wherein said HMG Co-A reductase inhibitor is selected from the group consisting of mevastatin, lovastatin, simvastatin, pravastatin, fluvastatin, cerivastatin, atorvastatin, rosuvastatin, pitavastatin, and the pharmaceutically acceptable salts, esters, conjugate acids, and prodrugs thereof.

16. Use according to Claim 13 or Claim 14 wherein said HMG Co-A reductase inhibitor is atorvastatin or a pharmaceutically acceptable salt, ester, conjugate acid, or prodrug thereof.

17. Use according to any one of Claims 13 to 16 wherein said eplerenone and said HMG Co-A reductase inhibitor are administered in a weight ratio range from about ten-to-one to about one-to-two of said eplerenone to said HMG Co-A reductase inhibitor.

18. Use according to Claim 17 wherein said weight ratio range is from about five-to-one to about one-to-one.

19. Use according to Claim 17 wherein said weight ratio range is from about two-to-one to about one-to-one.

20. Use according to any one of Claims 13 to 19 wherein said HMG Co-A reductase inhibitor is present in an amount between about 0.05 mg to about 100 mg.

21. Use according to any one of Claims 13 to 20 wherein said eplerenone is present in an amount between about 0.75 mg to about 200 mg.

## Patentansprüche

1. Kombination, umfassend eine erste Menge an Eplerenon und eine zweite Menge eines HMG-Co-A-Reduktase-Inhibitors.

2. Kombination nach Anspruch 1, wobei die Kombination eine kombinierte Dosierungsform von Eplerenon und eines HMG-Co-A-Reduktase-Inhibitors ist.

3. Kombination nach Anspruch 1, wobei die Kombination ein Kit ist, umfassend Eplerenon und einen HMG-Co-A-Reduktase-Inhibitor.

4. Kombination nach Anspruch 1, wobei die Kombination in Form einer pharmazeutischen Zusammensetzung vorliegt, die außerdem einen pharmazeutisch akzeptablen Träger umfaßt.

5. Kombination nach Anspruch 4, wobei die erste Menge und die zweite Menge zusammen eine therapeutisch wirksame Menge des Eplerenons und HMG-Co-A-Reduktase-Inhibitors umfassen.

6. Kombination nach einem der Ansprüche 1 bis 5, wobei der HMG-Co-A-Reduktase-Inhibitor ausgewählt ist aus der Gruppe, bestehend aus Mevastatin, Lovastatin, Simvastatin, Pravastatin, Fluvastatin, Cerivastatin, Atorvastatin, Rosuvastatin, Pitavastatin und den pharmazeutisch akzeptablen Salzen, Estern, korrespondierenden Säuren und Prodrugs davon.

7. Kombination nach einem der Ansprüche 1 bis 5, wobei der HMG-Co-A-Reduktase-Inhibitor Atorvastatin oder ein pharmazeutisch akzeptables Salz, Ester, korrespondierende Säure oder Prodrug davon ist.

8. Kombination nach einem der Ansprüche 1 bis 7, wobei das Eplerenon und der HMG-Co-A-Reduktase-Inhibitor in der Zusammensetzung in einem Gewichtsverhältnisbereich von etwa zehn zu eins bis etwa eins zu zwei des Eplerenons zu dem HMG-Co-A-Reduktase-Inhibitor vorliegen.

9. Kombination nach Anspruch 8, wobei der Gewichtsverhältnisbereich etwa fünf zu eins bis etwa eins zu eins beträgt.

10. Kombination nach Anspruch 8, wobei der Gewichtsverhältnisbereich etwa zwei zu eins bis etwa eins zu eins beträgt.

11. Kombination nach einem der Ansprüche 1 bis 10, wobei die zweite Menge des HMG-Co-A-Reduktase-Inhibitors zwischen etwa 0,05 mg und etwa 100 mg liegt.

12. Kombination nach einem der Ansprüche 1 bis 11, wobei die erste Menge des Eplerenons zwischen etwa 0,75 mg und etwa 200 mg liegt.

13. Verwendung von Eplerenon und eines HMG-Co-A-Reduktase-Inhibitors für die Herstellung eines Medikaments zur gleichzeitigen oder aufeinanderfolgenden Behandlung oder Vorbeugung eines kardiovaskulären Zustandes.

14. Verwendung nach Anspruch 13, wobei der kardiovaskuläre Zustand ausgewählt ist aus der Gruppe, bestehend aus Atherosklerose, Hypertonie, Herzversagen, Gefäßerkrankung, Nierendysfunktion, Schlaganfall, Myokardinfarkt, Endotheldysfunktion, ventrikulärer Hypertrophie, Zielorganschäden, Thrombose, Herzarrhythmie, Plaqueruptur und Aneurysma.

15. Verwendung nach Anspruch 13 oder Anspruch 14, wobei der HMG-Co-A-Reduktase-Inhibitor ausgewählt ist aus der Gruppe, bestehend aus Mevastatin, Lovastatin, Simvastatin, Pravastatin, Fluvastatin, Cerivastatin, Atorvastatin, Rosuvastatin, Pitavastatin und den pharmazeutisch akzeptablen Salzen, Estern, korrespondierenden Säuren und Prodrugs davon.

16. Verwendung nach Anspruch 13 oder Anspruch 14, wobei der HMG-Co-A-Reduktase-Inhibitor Atorvastatin oder ein pharmazeutisch akzeptables Salz, Ester, korrespondierende Säure oder Prodrug davon ist.

17. Verwendung nach einem der Ansprüche 13 bis 16, wobei das Eplerenon und der HMG-Co-A-Reduktase-Inhibitor in einem Gewichtsverhältnisbereich von etwa zehn zu eins bis etwa eins zu zwei des Eplerenons zu dem HMG-Co-A-Reduktase-Inhibitor verabreicht werden.

18. Verwendung nach Anspruch 17, wobei der Gewichtsverhältnisbereich von etwa fünf zu eins bis etwa eins zu eins beträgt.

19. Verwendung nach Anspruch 17, wobei der Gewichtsverhältnisbereich von etwa zwei zu eins bis etwa eins zu eins beträgt.

20. Verwendung nach einem der Ansprüche 13 bis 19, wobei der HMG-Co-A-Reduktase-Inhibitor in einer Menge zwischen etwa 0,05 mg und etwa 100 mg vorliegt.

21. Verwendung nach einem der Ansprüche 13 bis 20, wobei das Eplerenon in einer Menge zwischen etwa 0,75 mg und etwa 200 mg vorliegt.

## Revendications

1. Association comprenant une première quantité d'éplérénone et une seconde quantité d'un inhibiteur de HMG-Co-A-réductase.

2. Association suivant la revendication 1, l'association étant une forme posologique combinée d'éplérénone et d'un inhibiteur de HMG-Co-A-réductase.

3. Association suivant la revendication 1, l'association étant un kit comprenant de l'éplérénone et un inhibiteur de HMG-Co-A-réductase.

4. Association suivant la revendication 1, l'association étant sous forme d'une composition pharmaceutique comprenant en outre un support pharmaceutiquement acceptable.

5. Association suivant la revendication 4, dans laquelle ladite première quantité et ladite seconde quantité constituent conjointement une quantité thérapeutiquement efficace de ladite éplérénone et dudit inhibiteur de HMG-Co-A-réductase.

6. Association suivant l'une quelconque des revendications 1 à 5, dans laquelle ledit inhibiteur de HMG-Co-A-réductase est choisi dans le groupe consistant en la mévastatine, la lovastatine, la simvastatine, la pravastatine, la fluvastatine, la cérivastatine, l'atorvastatine, la rosuvastatine, la pitavastatine et leurs sels, esters, acides conjugués et précurseurs médicamenteux pharmaceutiquement acceptables.

7. Association suivant l'une quelconque des revendications 1 à 5, dans laquelle ledit inhibiteur de HMG-Co-A-réductase est l'atorvastatine ou un de ses sels, esters, acides conjugués ou précurseurs médicamenteux pharmaceutiquement acceptables.

8. Association suivant l'une quelconque des revendications 1 à 7, dans laquelle ladite éplérénone et ledit inhibiteur de HMG-Co-A-réductase sont présents dans ladite composition dans un intervalle de rapports pondéraux d'environ dix-à-un à environ un-à-deux de ladite éplérénone audit inhibiteur de HMG-Co-A-réductase.

9. Association suivant la revendication 8, dans laquelle ledit intervalle de rapports pondéraux va d'environ cinq-à-un à environ un-à-un.

10. Association suivant la revendication 8, dans laquelle ledit intervalle de rapports pondéraux va d'environ deux-à-un à environ un-à-un.

11. Association suivant l'une quelconque des revendications 1 à 10, dans laquelle ladite seconde quantité dudit inhibiteur de HMG-Co-A-réductase est comprise entre environ 0,05 mg et environ 100 mg.

12. Association suivant l'une quelconque des revendications 1 à 11, dans laquelle ladite première quantité de ladite éplérénone est comprise entre environ 0,75 mg et environ 200 mg.

13. Utilisation d'éplérénone et d'un inhibiteur de HMG-Co-A-réductase pour la production d'un médicament destiné au traitement ou à la prévention, de manière simultanée ou séquentielle, d'un trouble cardiovasculaire.

14. Utilisation suivant la revendication 13, dans laquelle ledit trouble cardiovasculaire est choisi dans le groupe consistant en l'athérosclérose, l'hypertension, l'insuffisance cardiaque, une maladie vasculaire, un dysfonctionnement rénal, un ictus, l'infarctus du myocarde, un dysfonctionnement endothélial, l'hypertrophie ventriculaire, l'altération d'un organe cible, une thrombose, l'arythmie cardiaque, la rupture de plaques et un anévrisme.

15. Utilisation suivant la revendication 13 ou la revendication 14, dans laquelle ledit inhibiteur de HMG-Co-A-réductase est choisi dans le groupe consistant en la mévastatine, la lovastatine, la simvastatine, la pravastatine, la fluvastatine, la cérivastatine, l'atorvastatine, la rosuvastatine, la pitavastatine et leurs sels, esters, acides conjugués et précurseurs médicamenteux pharmaceutiquement acceptables.

16. Utilisation suivant la revendication 13 ou la revendication 14, dans laquelle ledit inhibiteur de HMG-Co-A-réductase est l'atorvastatine ou un de ses sels, esters, acides conjugués ou précurseurs médicamenteux pharmaceutiquement acceptables.

17. Utilisation suivant l'une quelconque des revendications 13 à 16, dans laquelle ladite éplérénone et ledit inhibiteur de HMG-Co-A-réductase sont administrés dans un intervalle de rapports pondéraux d'environ dix-à-un à environ un-à-deux de ladite éplérénone audit inhibiteur de HMG-Co-A-réductase.

18. Utilisation suivant la revendication 17, dans laquelle ledit intervalle de rapports pondéraux va d'environ cinq-à-un à environ un-à-un.

19. Utilisation suivant la revendication 17, dans laquelle ledit intervalle de rapports pondéraux va d'environ deux-à-un à environ un-à-un.

20. Utilisation suivant l'une quelconque des revendications 13 à 19, dans laquelle ledit inhibiteur de HMG-Co-A-réductase est présent en une quantité comprise entre environ 0,05 mg et environ 100 mg.

21. Utilisation suivant l'une quelconque des revendications 13 à 20, dans laquelle ladite éplérénone est présente en une quantité comprise entre environ 0,75 mg et environ 200 mg.
